# EUROPEAN PATENT SPECIFICATION

(11) **EP 2 444 115 B1**
(45) Date of publication and mention of the grant of the patent: **25.12.2013**
(21) Application number: 11173678.1
(22) Date of filing: 12.07.2011
(51) Int. Cl.: A61M 25/00, A61M 29/00

(54) **Catheter assembly**
Katheteranordnung
Assemblage de cathéter

(30) Priority: 19.10.2010 JP 2010234956
(43) Date of publication of application: 25.04.2012
(73) Proprietor: ASAHI INTECC CO., LTD., Nagoya-shi, Aichi 463-0024 (JP)
(72) Inventor: Kajii, Tatsuhiko, Nagoya-shi, Aichi 463-0024 (JP)
(74) Representative: Winter, Brandl, Fürniss, Hübner, Röss, Kaiser, Polte - Partnerschaft

(56) References cited:
- EP-A1- 1 340 516
- WO-A1-94/03229
- WO-A1-2008/134382
- US-A1- 2004 103 516

## Description

### Technical Field

The present invention relates to a catheter assembly having a dilator and a catheter.

### Background Art

Conventionally, an assistive device called a sheath, into which a catheter used for diagnosis and treatment is inserted, is used to insert the catheter into a vessel (see, for example, JP-A-9-225035, JP-A-6-335531, and JP-A-6-178814). The sheath is inserted into a vessel in advance before the catheter is inserted into the vessel. The catheter is then inserted into the vessel by being inserted into the sheath.

In such an operation, as large a hole as an outer diameter of the sheath needs to be bored in the vessel. Recently, therefore, there has been proposed a catheter assembly enabling a catheter to be inserted into a vessel without using a sheath (see, for example, JP-A-2002-143318, and JP-A-2002-143319).

Such a catheter assembly has a catheter for diagnosis/treatment (hereinafter simply referred to as "catheter") such as a guiding catheter used for diagnosis or treatment inside a body, and a dilator to be inserted into the catheter. The dilator has a protruding portion which protrudes from a front end of the catheter and is inserted into a vessel. In the catheter assembly having the above configuration, the protruding portion of the dilator is inserted into the vessel to guide the catheter into the vessel together with the dilator. The catheter assembly is inserted by a predetermined length into a body, and then the dilator is pulled out of the body leaving the catheter behind.

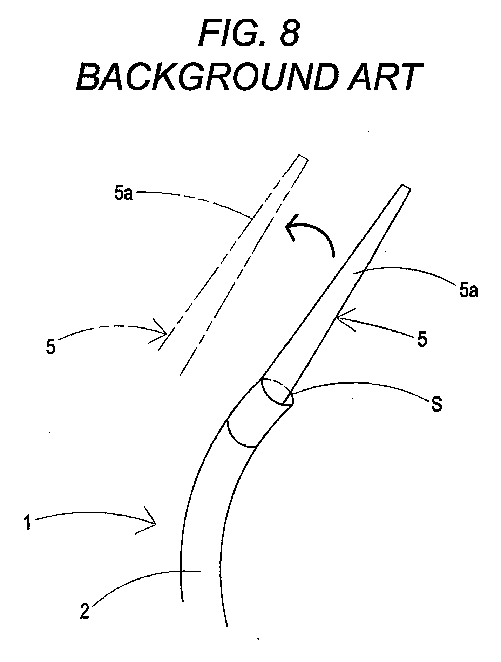

As illustrated in Fig. 8, a catheter 1 constituting such a catheter assembly may have various shapes such as of a curved portion 2 for suitably conducting diagnosis or treatment inside the body. A dilator 5, on the other hand, is not used at the time of diagnosis or treatment. Therefore, the dilator 5 is typically a substantially straight tubular member. In addition, the dilator 5 has a protruding portion 5a which protrudes from a front end of the catheter 1 to be inserted into a vessel. The protruding portion 5a is formed of a resin material harder than a resin material of the catheter 1. The protruding portion 5a thus has a higher stiffness than the catheter 1.

As a result, when the dilator 5 is inserted into the catheter 1 having the curved portion 2, the dilator 5 is curved along the curved portion 2 of the catheter 1. In this case, the dilator 5 tries to return to its straight shape, as shown by the two-dot chain line. A repulsive force is thus generated in the dilator 5. As a result, a central axis of the protruding portion 5a of the dilator 5 may be shifted from a central axis of an opening of the catheter 1, at the opening at the front end of the catheter 1. In this case, a stepped portion S is formed between the opening of the catheter 1 and the protruding portion 5a of the dilator 5.

Such a stepped portion S may inhibit the front end of the catheter 1 from being inserted into the body along the protruding portion 5a of the dilator 5.

A catheter assembly in accordance with the preamble of claim 1 is disclosed in WO 2008/134382 A1. In particular, the catheter assembly disclosed in WO 2008/134382 A1 comprises a sinus guide catheter which is substantially rigid and has a distal curved portion, and a dilator configured to be inserted into the catheter and comprising an elongate shaft having a proximal shaft section that is substantially rigid and a distal shaft section that is more flexible than the proximal shaft section. An expandable dilator, such as a balloon, is mounted to the distal shaft section and a relatively flexible distal tip member protrudes beyond the distal end thereof.

### Summary of Invention

The present invention has been made in view of the above described circumstances. Starting from a catheter assembly as known from WO 2008/134382 A1, an object of the invention is to provide a catheter assembly including a catheter having a curved portion and a dilator to be inserted into the catheter, wherein it is possible to prevent formation of a stepped portion between an opening of the catheter and a protruding portion of the dilator, and thus to smoothly insert the catheter into a body.

This object is achieved by a catheter assembly in accordance with claim 1. Preferred embodiments are subject-matter of dependent claims. Preferred embodiments of the catheter assembly are subject-matter of dependent claims.

### Brief Description of Drawings

The foregoing and other objects, features, aspects and advantages of the invention will become more apparent from the following detailed description when taken in conjunction with the accompanying drawings.
Fig. 1 is a view illustrating an entire catheter of a catheter assembly according to a first embodiment.
Fig. 2 is a view illustrating an entire dilator of the catheter assembly according to the first embodiment.
Fig. 3 is a view illustrating the catheter assembly according to the first embodiment in which the dilator has been inserted into the catheter.
Figs. 4A and 4B are enlarged views illustrating front end portions of the dilator and the catheter, respectively, according to the first embodiment.
Fig. 5 is an explanatory drawing of function of the first embodiment.
Figs. 6A and 6B are views illustrating a catheter assembly according to a second embodiment.
Figs. 7A and 7B are views illustrating a catheter assembly according to a third embodiment.
Fig. 8 is an explanatory drawing of the background art.

### Description of Embodiments

Preferred embodiments of the present invention will be described below with reference to the accompanying drawings, in which like reference characters designate similar or identical parts throughout the several views thereof.
<1> In the catheter assembly according to claim 1, the low stiffness portion of the dilator is arranged on the rear side of the protruding portion and at a position corresponding to the curved portion of the catheter and has a lower flexural stiffness than the protruding portion. Further, the protruding portion has a tapered portion on its front and a straight portion on the rear side of the tapered portion wherein, when the dilator is inserted into the catheter, the tapered portion and a front side portion of the straight portion protrude from a front end of the catheter and a rear side portion of the straight portion is positioned inside the catheter, and a flexural stiffness of the straight portion is higher than that of the low stiffness portion. With this configuration, the repulsive force of the dilator to return to its original shape is small. Consequently, the dilator is curved along the curved portion of the catheter. This prevents, as much as possible, the central axis of the protruding portion of the dilator from being shifted from the central axis of the opening of the catheter. As a result, the stepped portion is prevented from being formed between the front end of the catheter and the protruding portion of the dilator. Therefore, the catheter assembly can be smoothly inserted into a body.

Also in the catheter assembly according to claim 1, the high stiffness portion of the dilator is arranged on the rear side of the low stiffness portion and has a higher flexural stiffness than the low stiffness portion. With this configuration, the portion of the catheter on the rear side of the curved portion has a high straightness by virtue of the high stiffness portion of the dilator inserted into the catheter. Therefore, the catheter assembly can be smoothly inserted into the body. Even after inserted into the body, the catheter assembly can be smoothly advanced toward the target site inside the body.
<2> According to claim 2, the low stiffness portion of the dilator is arranged at a position corresponding to the first curved portion, which is at the most distal side among the plurality of curved portions of the catheter. With this configuration, the repulsive force of the dilator to return to its original shape is small at the curved portion closest to the front end of the catheter. Consequently, the dilator is curved along the curved portion of the catheter. This further effectively prevents the central axis of the protruding portion of the dilator from being shifted from the central axis of the opening of the catheter. As a result, the stepped portion is effectively prevented from being formed between the front end of the catheter and the protruding portion of the dilator. Therefore, the catheter assembly can be smoothly inserted into the body.

Also according to claim 2, the high stiffness portion of the dilator is arranged at a position corresponding to the second curved portion, which is on the rear side of the first curved portion of the catheter. With this configuration, the curve of the curved portion on the rear side is relieved by the repulsive force of the high stiffness portion of the dilator to return to its original shape. This makes the shape of the curved portion close to straight. Therefore, the catheter assembly can be smoothly inserted into the body. Even after inserted into the body, the catheter assembly can be smoothly advanced toward the target site inside the body.
<3> According to claim 3, the outer diameter of the low stiffness portion of the dilator is smaller than those of both the protruding portion and the high stiffness portion. Therefore, the respective portions of the dilator can be made of the same material. This makes it easy to form the protruding portion, the low stiffness portion, and the high stiffness portion of the dilator.

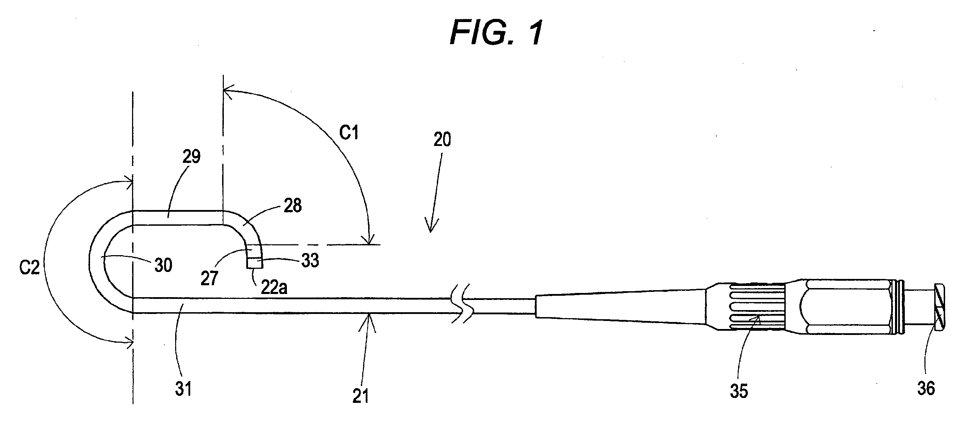

A catheter assembly according to a first embodiment will be described with reference to Figs. 1 to 5.

In Figs. 1 to 3, 4A and 4B, the left side shown is the front side (distal side) to be inserted into a body, and the right side is the rear side (proximal side, base end side) to be operated by an operator such as a physician.

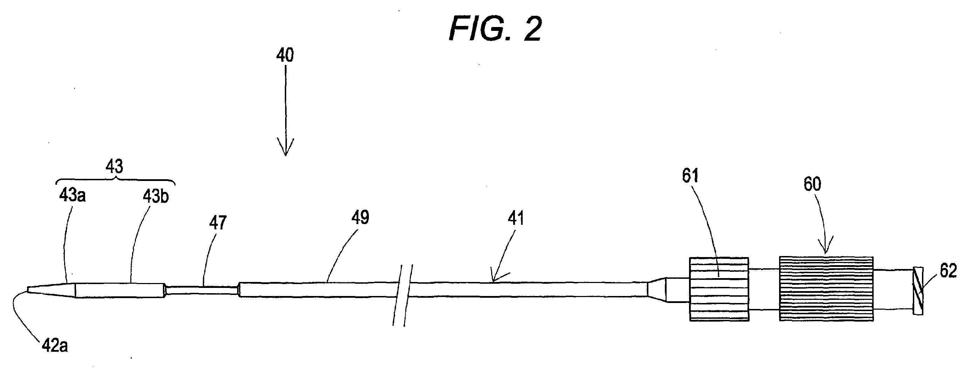

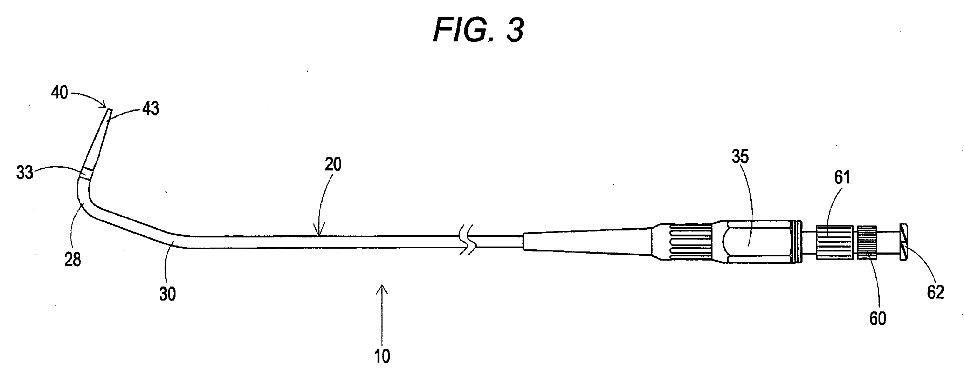

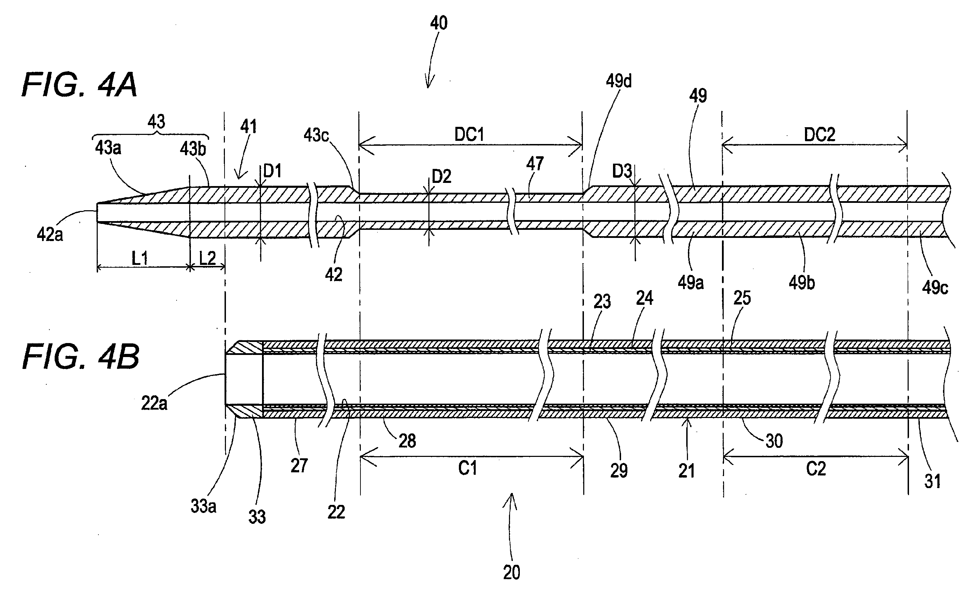

The entire length of a catheter assembly 10 is about 800 mm to about 1500 mm. The catheter assembly 10 includes, as illustrated in Fig. 3, a catheter 20 for diagnosis/treatment and a dilator 40. Fig. 1 illustrates the catheter 20, and Fig. 2 illustrates the dilator 40. The catheter 20 is used as a guiding catheter for guiding, for example, a balloon catheter which is used to treat a stenosis of a vessel in the heart or the like. Fig. 3 illustrates the dilator 40 inserted into the catheter 20. Figs. 4A and 4B illustrate a positional relationship between structural elements of the dilator 40 and structural elements of the catheter 20 when the dilator 40 is inserted into the catheter 20. In the catheter 20 illustrated in Fig. 4B, two curved portions 28 and 30 described later are kept straight for easy understanding. Also for easy understanding, dimensions of some structural elements are illustrated in an exaggerated manner.

The catheter 20 is a tubular member having a circular cross section, and includes therein a lumen 22. The catheter 20 mainly includes a catheter shaft 21, a tip 33, and a connector 35.

As illustrated in Fig. 4B, the catheter shaft 21 includes an inner layer 23 made of resin and constituting most of the lumen 22. A braid 24 is arranged on the outer surface of the inner layer 23. Furthermore, an outer layer 25 made of resin is arranged on the outer surface of the braid 24. Examples of the resin material of the inner layer 23 and the outer layer 25 of the catheter shaft 21 include polyamide, polyester, and polyurethane. The braid 24 has a known configuration in which a metal wire such as stainless steel is wound in the form of mesh.

As described above, the catheter 20 is a guiding catheter in the present embodiment. The catheter 20 illustrated in Fig. 1 is an example of the Judkins left coronary guiding catheter. However, the shape of the catheter 20 is not limited to that of the Judkins left coronary catheter. The catheter 20 may be a Judkins right coronary catheter or an Amplatz left or right coronary catheter, for example, as long as it has at least one curved portion.

The catheter shaft 21 of the Judkins left coronary catheter according to the first embodiment has two curved portions. That is, the catheter shaft 21 has a first straight shaft portion 27, the first curved portion 28, a second straight shaft portion 29, the second curved portion 30, and a body shaft portion 31 in this order from the front end of the catheter shaft 21.

The first straight shaft portion 27 is a straight portion arranged on the rear side of the tip 33 described later.

The first curved portion 28 on the front one of the curved portions is arranged on the rear side of the first straight shaft portion 27. Fig. 1 illustrates a curving range C1 of the first curved portion 28. The rear end of the range C1 is set, for example, within about 30 mm of the front end of the catheter 20. When the dilator 40 is not inserted into the catheter 20, the catheter shaft 21 is curved at about 90 degrees at the first curved portion 28.

The second straight shaft portion 29 is a straight portion arranged on the rear side of the first curved portion 28.

The second curved portion 30 is arranged on the rear side of the second straight shaft portion 29. Fig. 1 illustrates a curving range C2 of the second curved portion 30. When the dilator 40 is not inserted into the catheter 20, the catheter shaft 21 is curved at about 180 degrees at the second curved portion 30.

The body shaft portion 31 is a substantially straight portion constituting the rest of the catheter shaft 21, that is, the portion on the rear side of the second curved portion 30.

The tip 33 is attached to the front end of the first straight shaft portion 27 of the catheter shaft 21. The tip 33 is a cylindrical member having a hollow portion constituting the front end portion of the lumen 22. The tip 33 has, at a front end thereof, an opening 22a of the lumen 22. The axial length of the tip 33 is about 3.0 mm. The tip 33 is made of the same resin material as that of the catheter shaft 21. However, the tip 33 may be made of a softer resin material than the resin material of the catheter shaft 21. The resin material of the tip 33 contains a radiopaque material, which makes it possible to locate the catheter 20 under radioscopy.

The tip 33 has, at the front end thereof, a tapered portion 33a tapering toward the front end. The tapered portion 33a is provided to minimize a stepped portion described later, which is formed between the front end of the tip 33 and a protruding portion 43 of the dilator 40.

Note that the outer surfaces of the catheter shaft 21 and the tip 33 of the catheter 20 are provided with hydrophilic coating.

The connector 35 is attached to the rear end of the catheter shaft 21. The connector 35 includes therein a hollow portion communicating with the lumen 22 of the catheter shaft 21. A connecting portion 36 is provided on the rear end of the connector 35. The connecting portion 36 is threadably engaged with a front attachment part 61, described later, of a connector 60 of the dilator 40.

The dilator 40 is a cylindrical member including therein a lumen 42. The dilator 40 mainly includes a dilator shaft 41 and the connector 60. As illustrated in Fig. 3, the entire dilator 40 is set longer than the entire catheter 20 by a predetermined length. With this configuration, the front end portion of the dilator 40 protrudes from the front end of the catheter 20 when the dilator 40 is inserted into the catheter 20 and the connector 60 of the dilator 40 is connected to the connector 35 of the catheter 20 (hereinafter simply expressed as "when the dilator 40 is attached into the catheter 20").

The dilator shaft 41 includes the protruding portion 43, a low stiffness portion 47, and a high stiffness portion 49 in this order from the front end of the dilator shaft 41. The dilator shaft 41 is made of a single resin material. Examples of the resin material include polyamide and polyester as well as fluorine resin such as PTFE.

The protruding portion 43 is a front end portion of the dilator shaft 41. The protruding portion 43 includes a tapered portion 43 a on the front of the protruding portion 43 and a straight portion 43b on the rear side of the tapered portion 43a.

The tapered portion 43a protrudes from the front end of the catheter 20 when the dilator 40 is attached into the catheter 20. The tapered portion 43a has an inclined shape with its outer diameter decreasing toward the front end of the tapered portion. An opening 42a of the lumen 42 is formed at the front end of the tapered portion 43a. With this configuration, the tapered portion 43a makes it easy for the catheter assembly 10 to be inserted into a body. An axial length L1 of the tapered portion 43a is set to about 20 mm to about 25 mm in the present embodiment.

The straight portion 43b is a cylindrical member having a constant outer diameter. A front side portion (portion of an axial length L2 from the front end) of the straight portion 43b (see Fig. 4A) protrudes from the front end of the catheter 20 when the dilator 40 is inserted into the catheter 20. The axial length L2 is set to about 30 mm or shorter, and is about 5.0 mm in the present embodiment. An outer diameter D1 of the straight portion 43b is set as large as possible in the range allowing insertion/removal of the dilator 40 into/from the lumen 22 of the catheter 20. This is for minimizing the stepped portion between the front end of the tip 33 of the catheter 20 and the protruding portion 43 of the dilator 40.

The rear side portion of the straight portion 43b excluding the front side portion having the length L2 is positioned inside the catheter 20 when the dilator 40 is attached into the catheter 20. A transition portion 43 c is formed at the rear end of the straight portion 43b. The transition portion 43c has a tapered shape tapering toward the rear end. With this configuration, the transition portion 43c smoothly connects the straight portion 43b and the low stiffness portion 47.

The low stiffness portion 47 corresponds to the curving range C1 of the first curved portion 28 of the catheter 20 when the dilator 40 is inserted into the catheter 20. When the dilator 40 is attached into the catheter 20, the dilator 40 normally tries to keep its straight shape against the curve of the first curved portion 28. This consequently makes the central axis of the protruding portion 43 shift from the central axis of the opening 22a of the catheter 20. The low stiffness portion 47 is for preventing such axis-shift and has a low flexural stiffness. Therefore, an axial length DC1 of the low stiffness portion 47 is preferably equal to or longer than the curving range C1 of the first curved portion 28. In the first embodiment, the axial length DC1 of the low stiffness portion 47 is approximately equal to the curving range C1 of the first curved portion 28.

An outer diameter D2 of the low stiffness portion 47 is set smaller than the outer diameter D1 of the straight portion 43b of the protruding portion 43. This is for reducing the flexural stiffness of the low stiffness portion 47. The low stiffness portion 47 preferably has such flexural stiffness as to allow the low stiffness portion 47 to be deformed along the curve of the first curved portion 28 of the catheter 20. Therefore, the outer diameter D2 of the low stiffness portion 47 is determined considering the flexural stiffness of the catheter shaft 21, the curving degree of the first curved portion 28 and the like, in addition to the flexural stiffness of the protruding portion 43.

The high stiffness portion 49 is at the rear side of the low stiffness portion 47. The high stiffness portion 49 has a constant outer diameter D3. The high stiffness portion 49 includes three portions, i.e., a front portion 49a, a middle portion 49b, and a rear portion 49c corresponding to the respective portions of the catheter 20 when the dilator 40 is attached to the catheter 20. That is, the middle portion 49b corresponds to the curving range C2 of the second curved portion 30 of the catheter 20 when the dilator 40 is attached into the catheter 20. The front portion 49a is at the front side of the middle portion 49b. The rear portion 49c is at the rear side of the middle portion 49b.

A transition portion 49d is formed at the front end of the front portion 49a of the high stiffness portion 49. The transition portion 49d has a tapered shape tapering toward the front end. With this configuration, the transition portion 49d smoothly connects the front portion 49a of the high stiffness portion 49 and the low stiffness portion 47.

The middle portion 49b of the high stiffness portion 49 is for making the curve of the second curved portion 30 close to straight. For this purpose, the middle portion 49b utilizes the repulsive force of the dilator 40, attached into the catheter 20, to return to its straight shape. Therefore, the flexural stiffness of the middle portion 49b is set higher than that of the low stiffness portion 47. Specifically, the outer diameter D3 of the middle portion 49b is equal to the outer diameter D1 of the straight portion 43b of the protruding portion 43. In this manner, the flexural stiffness of the middle portion 49b is increased by making the outer diameter D3 as large as possible.

In the first embodiment, all the three portions of the front portion 49a, the middle portion 49b, and the rear portion 49c have the same outer diameter. Actually, therefore, these three portions constitute one portion. When the three portions are assumed to be separate members, however, an axial length DC2 of the middle portion 49b is preferably equal to or longer than the curving range C2 of the second curved portion 30.

The rear portion 49c of the high stiffness portion 49 is at the rear side of the middle portion 49b. The rear portion 49c corresponds to the body shaft portion 31 of the catheter 20. The connector 60 is connected to the rear end of the rear portion 49c.

The connector 60 includes therein a hollow portion communicating with the lumen 42 of the dilator shaft 41. The front attachment part (rotating portion) 61, which is threadably engaged with the connecting portion 36 of the connector 35 of the catheter 20, is provided at the front end of the connector 60. A rear end opening 62 is provided at the rear end of the connector 60. A guidewire (not illustrated) inserted into the lumen 42 extends through the rear end opening 62.

Fig. 3 illustrates the dilator 40 attached into the catheter 20 having the above configuration. As illustrated in Fig. 3, the protruding portion 43 of the dilator 40 protrudes from the front end of the catheter 20.

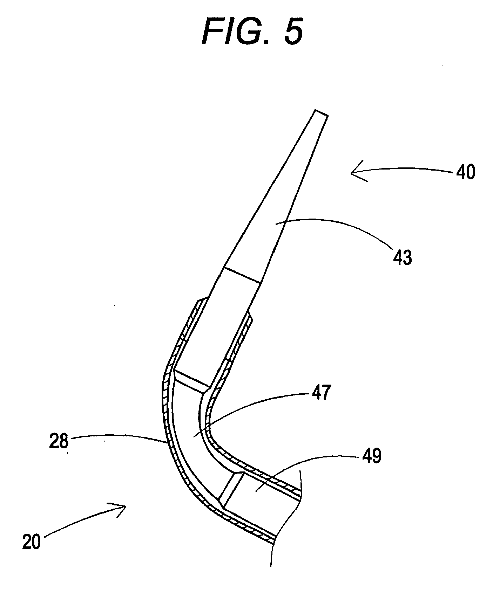

The low stiffness portion 47 of the dilator 40 corresponding to the curving range C1 of the first curved portion 28 of the catheter 20 has a low flexural stiffness. Consequently, the repulsive force of the dilator 40 to return to its straight shape is small at the low stiffness portion 47. As illustrated in Fig. 5, therefore, the low stiffness portion 47 of the dilator 40 is curved along the curve of the first curved portion 28. This prevents the axis of the straight portion 43b of the protruding portion 43 of the dilator 40 from being shifted from the axis of the first straight shaft portion 27 arranged on the front side of the first curved portion 28 as much as possible. This in turn prevents the stepped portion from being formed between the front end of the tip 33 and the protruding portion 43 of the dilator 40 at the opening 22a of the catheter 20 as much as possible.

The outer diameter D1 of the protruding portion 43 is set as large as possible in the range allowing the dilator 40 to be inserted into the lumen 22 of the catheter 20. This further prevents the stepped portion from being formed between the front end of the tip 33 of the catheter 20 and the protruding portion 43 of the dilator 40.

Furthermore, the tapered portion 33a is provided at the front end of the tip 33 of the catheter 20. Therefore, the front end of the tip 33 of the catheter 20 and the protruding portion 43 of the dilator 40 are smoothly connected.

In the catheter assembly 10 according to the first embodiment, as described above, the stepped portion is prevented from being formed between the front end of the tip 33 of the catheter 20 and the protruding portion 43 of the dilator 40. Furthermore, the front end of the tip 33 of the catheter 20 and the protruding portion 43 of the dilator 40 are smoothly connected. This configuration reduces insertion resistance generated when the front end of the dilator 40 and, subsequently, the front end of the catheter 20 are inserted into a body. Consequently, the catheter assembly 10 can be smoothly inserted into the body.

The middle portion 49b of the high stiffness portion 49 of the dilator 40 corresponding to the curving range C2 of the second curved portion 30 of the catheter 20 has a high stiffness. Therefore, the repulsive force of the dilator 40 to return to its straight shape makes the curved shape of the second curved portion 30 close to straight.

As a result, the second curved portion 30 is deformed into an approximately straight shape when the first curved portion 28 of the catheter 20 and, subsequently, the second curved portion 30 thereof are inserted into the body, and when the second curved portion 30 is pushed toward a target site inside the vessel. This makes it possible for the second curved portion 30 to be smoothly inserted into the body and then advance therein.

An operation for inserting the catheter assembly 10 of the first embodiment into an aorta of the heart will be described based on the above configuration. Note that, as described above, the catheter 20 of the first embodiment is the Judkins left coronary catheter. Consequently, the catheter 20 is engaged with the left coronary artery.

The catheter assembly 10 is inserted from a radial artery of a wrist region and into the aorta through a vessel in the vicinity of the upper arm and the collarbone, after which the dilator 40 is removed. With this operation, only the catheter 20 is engaged with a left coronary artery ostium. In this state, a treatment instrument (not illustrated) such as a treatment catheter (e.g., balloon catheter) is inserted into the lumen 22 of the catheter 20. This treatment instrument is used to treat a stenosis inside the coronary artery or the like.

To perform such an operation, first, a minute hole is bored in the radial artery of the wrist region with a puncture needle (not illustrated). The puncture needle typically has an outer cylinder into which the guidewire is inserted, and an inner cylinder inserted into the outer cylinder and provided with a needle body for puncture. The puncture needle with the inner cylinder inserted into the outer cylinder punctures the radial artery of the wrist region, whereby a minute hole is bored in the vessel. After that, only the inner cylinder is removed.

The guidewire (not illustrated) is inserted into the remaining outer cylinder. At this time, the guidewire is inserted up to the vicinity of the upper arm. Then, the outer cylinder of the puncture needle is removed, leaving only the guidewire behind. The catheter assembly 10 is then inserted from the rear end of the guidewire. At this time, the dilator 40 is already attached into the catheter 20 (as illustrated in Fig. 3).

The rear end of the guidewire is inserted through the opening 42a formed at the front end of the protruding portion 43 of the dilator 40, passes through the lumen 42, and extends through the rear end opening 62 of the connector 60. The catheter assembly 10 is then inserted into the body along the guidewire.

The front end of the protruding portion 43 of the dilator 40 is inserted into the body first. The front end portion of the protruding portion 43 is the tapered portion 43a. This minimizes the stepped portion between the front end of the dilator 40 and the outer surface of the guidewire. Consequently, the dilator 40 can be smoothly inserted into the body.

Subsequently, the front end portion of the catheter 20 is inserted into the body along the protruding portion 43 of the dilator 40. At this time, the outer diameter D1 of the protruding portion 43 is set as large as possible. This prevents the stepped portion from being formed between the front end of the tip 33 of the catheter 20 and the protruding portion 43 of the dilator 40. The tapered portion 33a is formed at the front end of the tip 33 of the catheter 20. With this configuration, the front end of the tip 33 of the catheter 20 and the protruding portion 43 of the dilator 40 are smoothly connected.

Furthermore, the low stiffness portion 47 of the dilator 40 corresponding to the first curved portion 28 of the catheter 20 has a small diameter. This reduces the flexural stiffness of the low stiffness portion 47. As a result, the repulsive force of the dilator 40 to return to its straight shape is small at the low stiffness portion 47. Consequently, the low stiffness portion 47 of the dilator 40 is curved along the curve of the first curved portion 28. This prevents the central axis of the straight portion 43b of the protruding portion 43 of the dilator 40 from being shifted from the central axis of the first straight shaft portion 27 arranged on the front side of the first curved portion 28. This in turn prevents the stepped portion from being formed between the front end of the tip 33 of the catheter 20 and the protruding portion 43 of the dilator 40 as much as possible.

As described above, the stepped portion is prevented from being formed between the front end of the tip 33 of the catheter 20 and the protruding portion 43 of the dilator 40, which are smoothly connected. As a result, the front end portion of the catheter 20 is smoothly inserted into the body along the protruding portion 43 of the dilator 40.

After the tip 33 of the catheter 20 is inserted into the body, the first straight shaft portion 27, the first curved portion 28, the second straight shaft portion 29, and the second curved portion 30 are sequentially inserted into the body.

The middle portion 49b of the high stiffness portion 49 of the dilator 40 corresponding to the second curved portion 30 of the catheter 20 has a high flexural stiffness. When inserted into the body, therefore, the second curved portion 30 is deformed to be close to straight by the repulsive force of the dilator 40 to return to its straight shape, as illustrated in Fig. 3.

When inserted into the body, therefore, the second curved portion 30 of the catheter 20 is unbent to be close to straight. As a result, the second curved portion 30 can be smoothly inserted into the body.

Subsequently, the body shaft portion 31 of the catheter 20 is inserted into the body along the guidewire. In this manner, the catheter assembly 10 is inserted into the vessel. Also when the catheter assembly 10 advances inside the vessel, the second curved portion 30 of the catheter 20 is deformed to be close to straight. This enables the catheter assembly 10 to move smoothly inside the vessel.

When the front end of the catheter assembly 10 approaches the front end of the guidewire positioned in the upper arm, an operator such as a physician moves the guidewire and the catheter assembly 10 at the same time. With this operation, the catheter assembly 10, with a predetermined length of the guidewire protruding from the front end thereof, passes through a vessel in the vicinity of the collarbone and reaches the vicinity of an inlet of an ascending aorta.

After that, the dilator 40 is pulled out of the catheter 20 and out of the body. After the dilator 40 is pulled out, the second curved portion 30 of the catheter 20 almost returns to its original shape. In this state, the operator operates the guidewire and the catheter 20 to advance these further toward the ascending aorta. Then, the operator also removes the guidewire out of the body, and engages the front end of the catheter 20 with the left coronary artery ostium.

Note that the timing of removing the dilator 40 and the guidewire depends on the operator, and is not limited to the above example.

When the catheter 20 is engaged with the coronary artery ostium in this manner, a treatment instrument such as another guidewire or balloon catheter is inserted into the catheter 20 to treat a target site, i.e., a stenosis inside the coronary artery or the like. This treatment instrument is then used for treatment of the heart.

In the first embodiment described above, the diameter of the low stiffness portion 47 is made smaller than those of the other portions in order to reduce the flexural stiffness of the low stiffness portion 47 of the dilator 40 corresponding to the first curved portion 28 of the catheter 20. However, the method for reducing the flexural stiffness of the low stiffness portion 47 is not limited to decreasing the diameter thereof.

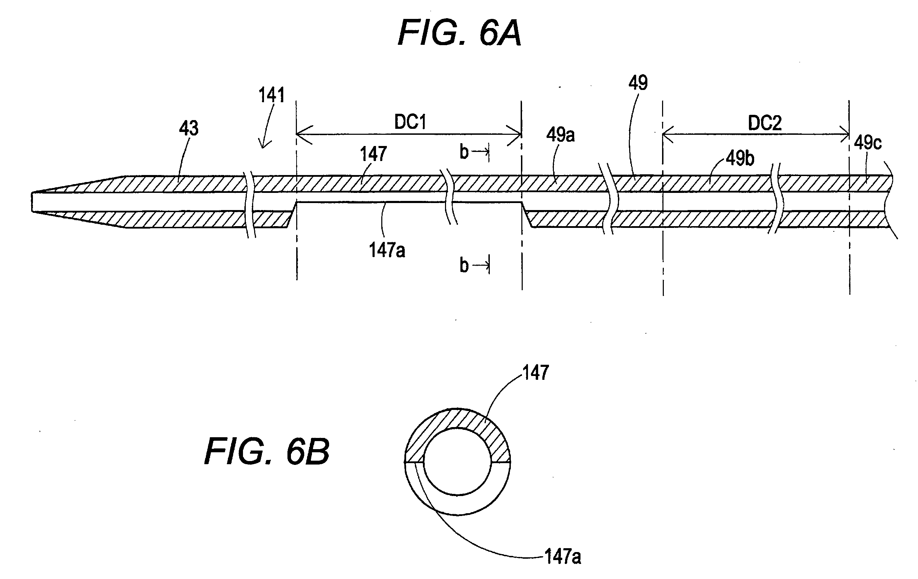

Like a dilator shaft 141 illustrated in Fig. 6A, for example, the dilator shaft may include a semicircular portion 147 having a semicircular cross section, instead of the low stiffness portion 47 described above. That is, as illustrated in Fig. 6B which is a cross-sectional view of the dilator shaft 141, the semicircular portion 147 is formed by cutting out substantially half the cross section of a cylindrical portion having the same outer diameter as the protruding portion 43 and forming a cutout portion 147a. With this configuration, the flexural stiffness of the semicircular portion 147 is low.

Cutting out only the half portion on one side of the cylinder in this manner makes it easy for the dilator shaft 141 to bend toward the side of the cutout portion 147a. That is, by positioning the cutout portion 147a of the semicircular portion 147 serving as the low stiffness portion inside the first curved portion 28 of the catheter 20, the semicircular portion 147 can easily be bent along the first curved portion 28 of the catheter 20.

Alternatively, the portion corresponding to the low stiffness portion can be formed of a flexible resin or a metallic coil.

In the embodiments described above, the diameter of the middle portion 49b of the high stiffness portion 49 of the dilator 40 is the same as those of the protruding portion 43 and the front portion 49a and the rear portion 49c of the high stiffness portion 49, in order to increase the flexural stiffness of the middle portion 49b corresponding to the second curved portion 30 of the catheter 20. However, the middle portion 49b only needs to have such flexural stiffness as to facilitate the insertion of the catheter assembly 10 by relieving the curve of the second curved portion 30 of the catheter 20. Therefore, the diameter of the middle portion 49b does not have to be the same as that of the protruding portion 43 or the like.

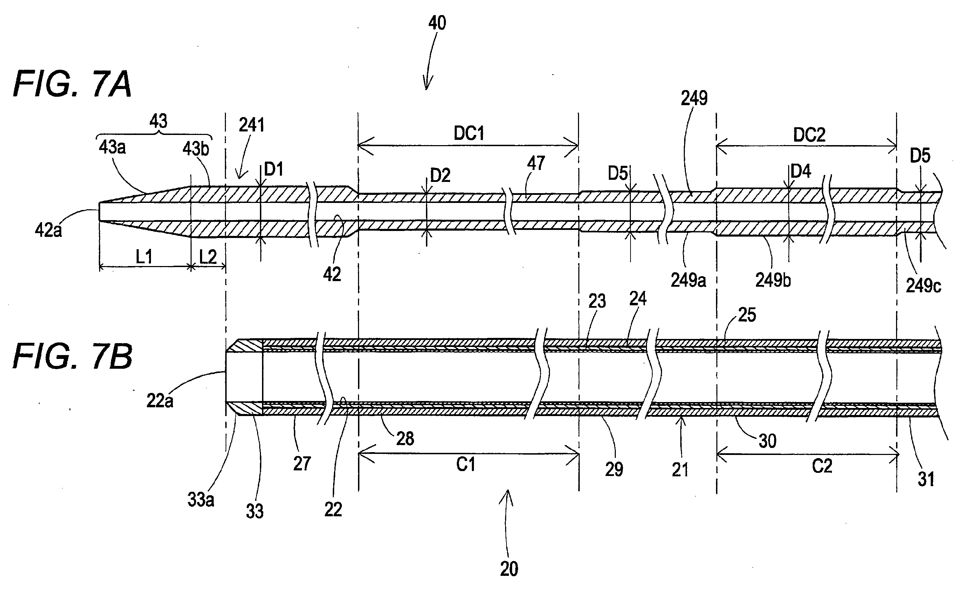

For example, a high stiffness portion 249 of a dilator shaft 241 illustrated in Figs. 7A and 7B may be used instead of the high stiffness portion 49 of the dilator 40 described above. That is, in the high stiffness portion 249, an outer diameter D4 of a middle portion 249b of the high stiffness portion 249 of a dilator 240 is smaller than the outer diameter D1 of the protruding portion 43 but larger than outer diameters D5 of a front portion 249a and a rear portion 249c of the high stiffness portion 249. The middle portion 249b corresponds to the second curved portion 30 of the catheter 20.

The middle portion 49b of the high stiffness portion 49 of the dilator 40 may be made of a hard resin to increase the flexural stiffness of the middle portion 49b.

In the embodiments described above, the catheter 20 of the catheter assembly 10 has been described as the guiding catheter for a heart. However, the catheter 20 is not limited to the above example and can be applied to other catheters.

In addition, the organ for which the catheter is used is not limited to a vessel in the heart. The catheter 20 can be used in an operation for other organs.

## Claims

1. A catheter assembly comprising:
a catheter (20) comprising a tubular member, and
at least one curved portion (28, 30) at a front portion of the tubular member; and
a dilator (40) configured to be inserted into the catheter (20), the dilator (40) comprising
a protruding portion (43) arranged on a front end portion of the dilator (40), at least part of the protruding portion (43) protruding from a front end of the catheter (20) when the dilator (40) is inserted into the catheter (20),
a low stiffness portion (47) arranged on a rear side of the protruding portion (43) and at a position corresponding to the curved portion of the catheter (20), and having a lower flexural stiffness than the protruding portion (43), and
a high stiffness portion (49) arranged on a rear side of the low stiffness portion (47) and having a higher flexural stiffness than the low stiffness portion (47), **characterized in that**:
the protruding portion (43) has a tapered portion (43a) on its front and a straight portion (43b) on the rear side of the tapered portion (43a) wherein, when the dilator (40) is inserted into the catheter (20), the tapered portion (43a) and a front side portion of the straight portion (43b) protrude from a front end of the catheter (20) and a rear side portion of the straight portion (43b) is positioned inside the catheter (20), and
a flexural stiffness of the straight portion (43b) is higher than that of the low stiffness portion 47).

2. The catheter assembly according to claim 1, wherein the catheter (20) has a plurality of curved portions (28, 30),
the low stiffness portion (47) of the dilator (40) is arranged at a position corresponding to a first curved portion (28), which is at a most distal side among the plurality of curved portions of the catheter (20), and
the high stiffness portion (49) of the dilator (40) is arranged at a position corresponding to a second curved portion (30), which is on a rear side of the first curved portion (28) of the catheter (20).

3. The catheter assembly according to claim 1 or 2, wherein an outer diameter of the low stiffness portion (47) is smaller than outer diameters of both the protruding portion (43) and the high stiffness portion (49).

4. The catheter assembly according to claim 1, wherein an axial length (DC1) of the low stiffness portion (47) is approximately equal to a curving range of the curved portion (28, 30).

5. The catheter assembly according to any of claims 1 to 4, wherein the low stiffness portion (47) has a cutout portion (147a).

## Patentansprüche

1. Katheteranordnung mit:
einem Katheter (20), der ein röhrenförmiges Element aufweist, und
wenigstens einem Biegeabschnitt (28, 30) an einem vorderen Abschnitt des röhrenförmigen Elements; und
einem Dilatator (40) zum Einführen in den Katheter (20), wobei der Dilatator (40) aufweist
einen hervorstehenden Abschnitt (43), der am vorderen Endabschnitt des Dilatators (40) angeordnet ist und, wenn der Dilatator (40) in den Katheter (20) eingeführt ist, wenigstens teilweise aus dem vorderen Ende des Katheters (20) hervor steht,
einen Abschnitt (47) niedriger Steifigkeit, der an der Rückseite des hervorstehenden Abschnitts (43) und an einer Stelle entsprechend dem Biegeabschnitt des Katheters (20) angeordnet ist und eine niedrigere Biegesteifigkeit hat als der hervorstehende Abschnitt (43), und
einen Abschnitt (49) hoher Steifigkeit, der an der Rückseite des Abschnitts (47) niedriger Steifigkeit angeordnet ist und eine höhere Biegesteifigkeit hat als der Abschnitt (47) niedriger Steifigkeit, **dadurch gekennzeichnet, dass**:
der hervorstehende Abschnitt (43) an seiner Vorderseite einen verjüngten Abschnitt (43a) und an der Rückseite des verjüngten Abschnitts (43a) einen geraden Abschnitt (43b) hat, wobei, wenn der Dilatator (40) in den Katheter (20) eingeführt ist, der verjüngte Abschnitt (43a) und ein vorderer Teil des geraden Abschnitts (43b) aus dem vorderen Ende des Katheters (20) hervor ragen und ein hinterer Teil des geraden Abschnitts (43b) im Katheter (20) angeordnet ist, und
die Biegesteifigkeit des geraden Abschnitts (43b) höher ist als diejenige des Abschnitts (47) niedriger Steifigkeit.

2. Katheteranordnung nach Anspruch 1, wobei der Katheter (20) eine Vielzahl von Biegeabschnitten (28, 30) aufweist,
der Abschnitt (47) niedriger Steifigkeit des Dilatators (40) an einer Position entsprechend einem ersten Biegeabschnitt (28) angeordnet ist, der von der Vielzahl von Biegeabschnitten des Katheters (20) am distalsten angeordnet ist, und
der Abschnitt (49) hoher Steifigkeit des Dilatators (40) an einer Position entsprechend einem zweiten Biegeabschnitt (30) angeordnet ist, der an der Rückseite des ersten Biegeabschnitts (28) des Katheters (20) angeordnet ist.

3. Katheteranordnung nach Anspruch 1 oder 2, wobei der Außendurchmesser des Abschnitts (47) niedriger Steifigkeit kleiner ist als die Außendurchmesser des hervorstehenden Abschnitts (43) und des Abschnitts (49) hoher Steifigkeit.

4. Katheteranordnung nach Anspruch 1, wobei die axiale Länge (DC1) des Abschnitts (47) niedriger Steifigkeit in etwa gleich dem Biegebereich des Biegeabschnitts (28, 30) ist.

5. Katheteranordnung nach einem der Ansprüche 1 bis 4, wobei der Abschnitt (47) niedriger Steifigkeit einen ausgesparten Abschnitt (147a) hat.

## Revendications

1. Assemblage de cathéter comprenant :
un cathéter (20) comprenant un organe tubulaire, et
au moins une portion incurvée (28, 30) à une portion avant de l'organe tubulaire ; et
un dilatateur (40) configuré pour être inséré dans le cathéter (20), le dilatateur (40) comprenant :
une portion faisant saillie (43) agencée sur une portion d'extrémité avant du dilatateur (40), au moins une partie de la portion faisant saillie (43) faisant saillie à partir d'une extrémité avant du cathéter (20) lorsque le dilatateur (40) est inséré dans le cathéter (20),
une portion de faible rigidité (47) agencée sur un côté arrière de la portion faisant saillie (43) et à une position correspondant à la portion incurvée du cathéter (20), et ayant une rigidité de flexion inférieure à celle de la portion faisant saillie (43), et
une portion de grande rigidité (49) agencée sur un côté arrière de la portion de faible rigidité (47) et ayant une rigidité de flexion supérieure à celle de la portion de faible rigidité (47),
**caractérisé en ce que** :
la portion faisant saillie (43) comporte une portion conique (43a) sur son côté avant et une portion droite (43b) sur le côté arrière de la portion conique (43a) dans lequel, lorsque le dilatateur (40) est inséré dans le cathéter (20), la portion conique (43a) et une portion de côté avant de la portion droite (43b) font saillie à partir d'une extrémité avant du cathéter (20) et une portion de côté arrière de la portion droite (43b) est positionnée à l'intérieur du cathéter (20), et
une rigidité de flexion de la portion droite (43b) est supérieure à celle de la portion de faible rigidité (47).

2. Assemblage de cathéter selon la revendication 1, dans lequel le cathéter (20) comporte une pluralité de portions incurvées (28, 30),
la portion de faible rigidité (47) du dilatateur (40) est agencée à une position correspondant à une première portion incurvée (28), qui se trouve sur le côté le plus distal parmi la pluralité de portions incurvées du cathéter (20), et
la portion de grande rigidité (49) du dilatateur (40) est agencée à une position correspondant à une deuxième portion incurvée (30), qui se trouve sur un côté arrière de la première portion incurvée (28) du cathéter (20).

3. Assemblage de cathéter selon la revendication 1 ou 2, dans lequel un diamètre extérieur de la portion de faible rigidité (47) est inférieur aux diamètres extérieurs de la portion faisant saillie (43) et de la portion de grande rigidité (49).

4. Assemblage de cathéter selon la revendication 1, dans lequel une longueur axiale (DC1) de la portion de faible rigidité (47) est approximativement égale à une plage de courbure de la portion incurvée (28, 30).

5. Assemblage de cathéter selon l'une quelconque des revendications 1 à 4, dans lequel la portion de faible rigidité (47) comporte une portion de découpe (147a) .
